Europäisches Patentamt

European Patent Office

Office européen des brevets

(19)

(11) **EP 1 228 705 B1**

(12) **EUROPÄISCHE PATENTSCHRIFT**

(45) Veröffentlichungstag und Bekanntmachung des Hinweises auf die Patenterteilung:
**02.08.2006 Patentblatt 2006/31**

(21) Anmeldenummer: **02001308.2**

(22) Anmeldetag: **18.01.2002**

(51) Int Cl.:
*A23L 1/30* (2006.01)   *A23L 1/09* (2006.01)
*A23J 3/16* (2006.01)   *A23L 1/275* (2006.01)
*A61K 31/52* (2006.01)   *A23K 1/16* (2006.01)
*A23P 1/06* (2006.01)   *A23L 1/303* (2006.01)

(54) **Verfahren zur Herstellung von Trockenpulvern eines oder mehrerer Carotinoide**

Process to produce dry powder of a carotenoid or several carotenoids

Procédé de préparation de poudres sèches d' un caroténoide ou plusieurs caroténoides

(84) Benannte Vertragsstaaten:
**AT BE CH CY DE DK ES FI FR GB GR IE IT LI LU MC NL PT SE TR**
Benannte Erstreckungsstaaten:
**RO SI**

(30) Priorität: **31.01.2001 DE 10104494**

(43) Veröffentlichungstag der Anmeldung:
**07.08.2002 Patentblatt 2002/32**

(73) Patentinhaber: **BASF Aktiengesellschaft**
**67056 Ludwigshafen (DE)**

(72) Erfinder:
• **Runge, Frank, Dr.**
**67159 Friedelsheim (DE)**
• **Lüddecke, Erik, Dr.**
**67112 Mutterstadt (DE)**
• **Pfeiffer, Angelika-Maria, Dr.**
**67134 Birkenheide (DE)**

(56) Entgegenhaltungen:
**EP-A- 0 966 960**     **WO-A-91/06292**
**DE-A- 4 424 085**     **US-A- 4 522 743**

EP 1 228 705 B1

**Beschreibung**

Die Erfindung betrifft ein Verfahren zur Herstellung von Trockenpulvern eines oder mehrerer Carotinoide, bevorzugt von Xanthophyll-haltigen Trockenpulvern, insbesondere von Xanthophyllen, ausgewählt aus der Gruppe, bestehend aus Astaxanthin, Canthaxanthin, Lutein, Zeaxanthin, Citranaxanthin und β-Apo-8'-Carotinsäureethylester.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxy- und/oder Oxogruppen vor. Typische Vertreter dieser Gruppe sind u.a. Astaxanthin, Canthaxanthin, Lutein und Zeaxanthin.

Zu den Sauerstoff-haltigen Carotinoiden zählen auch Citranaxanthin und β-Apo-8'-Carotinsäureethylester.

Sauerstoff-haltige Carotinoide sind in der Natur weit verbreitet und kommen u.a. im Mais (Zeaxanthin), in grünen Bohnen (Lutein), in Paprika (Capsanthin), in Eidottern (Lutein) sowie in Krebsen und Lachsen (Astaxanthin) vor, wobei sie diesen Nahrungsmitteln ihre charakteristische Färbung verleihen.

Diese sowohl synthetisch zugänglichen als auch aus natürlichen Quellen isolierbaren Polyene stellen für die Lebens- und Futtermittelindustrie sowie für den pharmazeutischen Bereich wichtige Farbkörper dar und sind, wie im Falle von Astaxanthin, Wirkstoffe mit Provitamin-A Aktivität beim Lachs.

Sowohl Carotine als auch Xanthophylle sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebens- und Futtermitteln entgegen, da die Substanzen in grobkristalliner Form nur schlechte Färbungsergebnisse liefern. Diese für die praktische Verwendung der Xanthophylle nachteiligen Effekte wirken sich insbesondere im wäßrigen Medium aus.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Carotinoide bzw. Xanthophylle und damit indirekt zu besseren Färbungseffekten z.B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 $\mu$m zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 $\mu$m.

Daneben existieren eine Reihe von kombinierten Emulgier-/Sprühtrocknungsverfahren, wie sie z.B. in DE-A-12 11 911 oder in EP-A-0 410 236 beschrieben sind.

EP-A-0 996 960 offenbart ein Granulat, das Zeaxanthin, Lactose und Gelatine enthält.

Gemäß der europäischen Patentschrift EP-B-0 065 193 erfolgt die Herstellung von feinverteilten, pulverförmigen Carotinoidpräparaten dadurch, daß man ein Carotinoid in einem flüchtigen, mit Wasser mischbaren organischen Lösungsmittel bei erhöhten Temperaturen, gegebenenfalls unter erhöhtem Druck löst, das Carotinoid durch Mischen mit einer wäßrigen Lösung eines Schutzkolloids ausfällt und anschließend sprühtrocknet.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

Bei den nach EP-B-0 065 193 hergestellten nanopartikulären Wirkstoffdispersionen von Xanthophyllen sind jedoch häufig folgende Phänomene zu beobachten.

Die wäßrigen, Xanthophyll-haltigen Wirkstoffdispersionen sind häufig, insbesondere bei der Aufkonzentration, kolloidal instabil. Durch Ausflockungen der Wirkstoffpartikel, die dabei teilweise sedimentieren, teilweise aufrahmen, ist eine weitere Überführung der Dispersion in ein Trockenpulver nicht mehr möglich.

Bei Xanthophyllen mit Carbonyl-Funktionen kann außerdem die als Schutzkolloid eingesetzte Gelatine vernetzen, so daß ein Gel entsteht, das nicht mehr redispergierbar ist und das ebenfalls nicht weiter in ein Trockenpulver überführt werden kann.

Somit können die hohen Anforderungen an Xanthophyll-haltigen Formulierungen bezüglich Farbwirkung und Bioverfügbarkeit aufgrund der geschilderten Problematik mit dem o.g. Verfahren nicht immer erfüllt werden.

Nachteilig an Gelatinen sind auch deren stark klebenden Eigenschaften. Mit den für flüssige Systeme üblichen Trocknungsmethoden wie die Sprühtrocknung oder Sprühwirbelbett-Trocknung kann es bei Verwendung von gelatinehaltigen Produkten zu Fadenbildung oder Verbackungen kommen.

Hinzu kommt eine immer geringer werdende Akzeptanz des Verbrauchers gegenüber Gelatine-haltigen Produkten.

In andere oft verwendete Schutzkolloide wie Gummi arabicum, Stärke, Dextrine, Pektin oder Tragant lassen sich häufig nur relativ geringe Konzentrationen von fettlöslichen Substanzen einbetten. Darüber hinaus stand insbesondere Gummi arabicum in der Vergangenheit infolge von Mißernten nicht immer und in ausreichender Qualität zur Verfügung.

Synthetische Kolloide wie Polyvinylpyrrolidon oder partialsynthetische Polymere wie Cellulosederivate zeigen ebenfalls eine begrenzte Emulgierkapazität und werden vor allem im Lebensmittelbereich nicht immer akzeptiert.

DE-A-44 24 085 beschreibt die Verwendung von teilabgebauten Sojaproteinen als Schutzkolloide für fettlösliche Wirkstoffe. Die hier offenbarten Sojaproteine weisen einen Abbaugrad von 0,1 bis 5 % auf. Die Farbstärke der mit diesen Schutzkolloiden hergestellten Formulierungen ist dabei nicht immer zufriedenstellend.

Aufgabe der vorliegenden Erfindung war es daher, Verfahren zur Herstellung von Carotinoid-haltigen Trockenpulvern, insbesondere von Trockenpulvern Sauerstoff-haltiger Carotinoide unter Verwendung von Schutzkolloiden vorzuschlagen, die die oben genannten Nachteile des Standes der Technik nicht aufweisen.

Diese Aufgabe wurde erfindungsgemäß gelöst mit einem Verfahren zur Herstellung von Trockenpulvern eines oder mehrerer Carotinoide durch

a) Dispergieren eines oder mehrerer Carotinoide in einer wäßrigen molekulardispersen oder kolloiddispersen Lösung eines Gemisches aus Lactose und einem Schutzkolloid und

b) Überführung der gebildeten Dispersion in ein Trockenpulver durch Abtrennung des Wassers und gegebenenfalls zusätzlich verwendeter Lösungsmittel und Trocknung, gegebenenfalls in Gegenwart eines Überzugsmaterials,

dadurch gekennzeichnet, daß man als Schutzkolloid im Verfahrensschritt a) mindestens ein Sojaprotein verwendet.

Erfindungsgemäß werden als Schutzkolloide ein oder mehrere verschiedene Sojaproteine verwendet. Bevorzugt sind dabei Sojaproteine, welche einen Abbaugrad ("DH": "degree of hydrolysis") von 0,1 bis 20 %, besonders bevorzugt 3 bis 12 %, ganz besonders bevorzugt 6 bis 9 % aufweisen. Der Abbaugrad "DH" ist folgendermaßen definiert:

$$DH = \frac{\text{Anzahl der gespaltenen Peptidbindungen}}{\text{Gesamtzahl der Peptidbindungen}} \times 100\,\%$$

Der Abbaugrad kann gemäß der sogenannten "pH-Stat-Methode" bestimmt werden, wie von C.F. Jacobsen et al. in "Methods of Biochemical Analysis", Vol. IV, S. 171-210, Interscience Publishers Inc., New York 1957, beschrieben.

Der Teilabbau erfolgt in der Regel enzymatisch, wobei als geeignete Enzyme Proteasen aus Pflanzen, Mikroorganismen, Pilzen oder tierische Proteasen in Betracht kommen. Vorzugsweise erfolgt der Teilabbau mit der pflanzlichen Protease Bromelain.

Als Sojaproteine werden in der Regel handelsübliche Sojaprotein-Isolate und -Konzentrate mit Proteingehalten von 70 bis 90 Gew.-% eingesetzt, wobei die restlichen 10 bis 30 Gew.-% mehr oder weniger undefinierte andere Pflanzenbestandteile darstellen. Als bevorzugt verwendete Sojaproteine seien in diesem Zusammenhang nicht gen-modifizierte Sojaproteine genannt.

Die Sojaprotein-Isolate werden in wäßrigem Medium mit dem Enzym inkubiert, vorzugsweise bei Temperaturen von 50 bis 70°C und pH-Werten von 7 bis 9. Das geeignete Verhältnis Protein zu Enzym kann im Einzelfall für den gewünschten Abbaugrad in für den Fachmann einfachen Laborversuchen ermittelt werden.

Die wäßrigen Sojaproteinhydrolysat-Lösungen werden in der Regel so hergestellt, daß der Proteingehalt 6 bis 10 Gew.-% beträgt.

Das gewichtsdurchschnittliche Molekulargewicht der erfindungsgemäß verwendeten, teilabgebauten Sojaproteine liegt im Bereich von 15000 bis 250000, bevorzugt von 25000 bis 220000, besonders bevorzugt von 50000 bis 200000, ganz besonders bevorzugt im Bereich von 120000 bis 180000.

Es ist auch möglich, in dem erfindungsgemäßen Verfahren Mischungen von teilabgebauten Sojaproteinen unterschiedlicher Abbaugrade oder Mischungen aus teilabgebauten und nichtabgebauten Sojaproteinen als Schutzkolloide zu verwenden. Bei diesen Mischungen liegen deren gewichtsdurchschnittliche Molekulargewichte ebenfalls in den oben genannten Bereichen.

Unter dem Begriff Dispergieren ist bevorzugt die Herstellung wäßriger Suspensionen sowie wäßriger Emulsionen zu verstehen. Besonders bevorzugt handelt es sich beim Dispergierschritt a) um die Herstellung einer Suspension eines oder mehrerer Carotinoide in einer wäßrigen molekulardispersen oder kolloiddispersen Lösung eines Gemisches aus Lactose und mindestens einem Sojaprotein, bei der die dispergierte Phase mindestens einen der Wirkstoffe als nanopartikuläre Teilchen enthält.

Eine bevorzugte Ausführungsform des o.g. Verfahrens ist dadurch gekennzeichnet, daß man die im Verfahrensschritt a) hergestellte Suspension vor der Überführung in ein Trockenpulver mahlt. In diesem Fall wird der Wirkstoff [das/die Carotinoid(e)] vor dem Mahlvorgang bevorzugt in kristalliner Form suspendiert.

Die Mahlung kann dabei in an sich bekannter Weise z.B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 100 μm, bevorzugt 0,2 bis 50 μm, besonders bevorzugt 0,2 bis 20 μm, ganz besonders bevorzugt 0,2 bis 5 μm, insbesondere 0,2 bis 0,8 μm aufweisen. Der Begriff D[4,3] bezeichnet den volumengewichteten mittleren Durchmesser (siehe Handbuch zu Malvern Mastersizer S, Malvern Instruments Ltd., UK).

Nähere Einzelheiten zur Mahlung und den dafür verwendeten Apparaturen finden sich u.a. in Ullmann's Encyclopedia of Industrial Chemistry, Sixth Edition, 1999, Electronic Release, Size Reduction, Kapitel 3.6.: Wet Grinding sowie in EP-A-0 498 824.

Eine ebenfalls bevorzugte Variante des erfindungsgemäßen Verfahrens ist dadurch gekennzeichnet, daß das Dispergieren in der Stufe a) folgende Schritte enthält:

$a_1$) Lösen eines oder mehrerer Carotinoide in einem mit Wasser mischbaren, organischen Lösungsmittel oder in einer Mischung aus Wasser und einem mit Wasser mischbaren, organischen Lösungsmittel oder

$a_2$) Lösen eines oder mehrerer Carotinoide in einem mit Wasser nicht mischbaren, organischen Lösungsmittel und

$a_3$) Mischen der nach $a_1$) oder $a_2$) erhaltenen Lösung mit einer wäßrigen molekulardispersen oder kolloiddispersen Lösung eines Gemisches aus Lactose und mindestens einem Sojaprotein, wobei die hydrophobe Phase des Carotinoids als nanodisperse Phase entsteht.

Je nach Art der verwendeten Lösungsmittel kann es sich bei der nanodispersen Phase im Schritt $a_3$) um feste Nanopartikel (Suspension) oder um Nanotröpfchen (Emulsion) handeln.

Die in der Stufe $a_1$) verwendeten wassermischbaren Lösungsmittel sind vor allem wassermischbare, thermisch stabile, flüchtige, nur Kohlenstoff, Wasserstoff und Sauerstoff enthaltenene Lösungsmittel wie Alkohole, Ether, Ester, Ketone und Acetale zu nennen. Zweckmäßig verwendet man solche Lösungsmittel, die mindestens zu 10% wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propylether, Tetrahydrofuran oder Aceton verwendet.

Der Begriff "ein mit Wasser nicht mischbares organisches Lösungsmittel" steht im Sinne der vorliegenden Erfindung für ein organisches Lösungsmittel mit einer Wasserlöslichkeit bei Normaldruck von weniger als 10%. Als mögliche Lösungsmittel kommen dabei u.a. halogenierte aliphatische Kohlenwasserstoffe, wie z.B. Methylenchlorid, Chloroform und Tetrachlorkohlenstoff, Carbonsäureester wie Dimethylcarbonat, Diethylcarbonat, Propylencarbonat, Ethylformiat, Methyl-, Ethyl- oder Isopropylacetat sowie Ether wie Methyl-tert. butylether in Frage. Bevorzugte, mit Wasser nicht mischbare organische Lösungsmittel sind die folgenden Verbindungen aus der Gruppe, bestehend aus Dimethylcarbonat, Propylencarbonat, Ethylformiat, Ethylacetat, Isopropylacetat und Methyl-tert. butylether.

Bei dem erfindungsgemäßen Verfahren handelt es sich bevorzugt um die Herstellung von Trockenpulvern von Sauerstoff-haltigen Carotinoiden, besonders bevorzugt um Verbindungen, ausgewählt aus der Gruppe, bestehend aus Astaxanthin, Canthaxanthin, Lutein, Zeaxanthin, Citranaxanthin und β-Apo-8'-Carotinsäureethylester.

Besonders bevorzugt ist das erfindungsgemäße Verfahren dadurch gekennzeichnet ist, daß man

a) Astaxanthin und/oder Canthaxanthin in einem mit Wasser mischbaren, organischen Lösungsmittel oder einer Mischung aus Wasser und einem mit Wasser mischbaren, organischen Lösungsmittel bei Temperaturen größer 30°C löst,

b) die erhaltene Lösung mit einer wäßrigen molekulardispersen oder kolloiddispersen Lösung eines Gemisches aus Lactose und einem teilabgebauten Sojaproteins mit einem Abbaugrad von 0,1 bis 20 % mischt und

c) die gebildete Dispersion in ein Trockenpulver überführt.

Ganz besonders bevorzugt handelt es sich hierbei um ein Verfahren zur Herstellung von Astaxanthin-haltigen Trockenpulvern.

Die Herstellung der o.g. Trockenpulver erfolgt vorteilhafterweise so, daß man mindestens eines der Carotinoide in einem mit Wasser mischbaren, organischen Lösungsmittel bei Temperaturen größer 30°C, vorzugsweise zwischen 50°C und 240°C, insbesondere 100°C bis 200°C, besonders bevorzugt 140°C bis 180°C, gegebenenfalls unter Druck, löst.

Da die Einwirkung hoher Temperaturen u.U. den gewünschten hohen all-trans Isomerenanteil herabsetzen kann, löst man das/die Carotinoid(e) möglichst rasch, beispielsweise im Sekundenbereich, z.B. in 0,1 bis 10 Sekunden, besonders bevorzugt in weniger als 1 Sekunde. Zur raschen Herstellung der moleculardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man anschließend direkt mit der gegebenenfalls gekühlten wäßrigen molekulardispersen oder kolloiddispersen Lösung des Gemisches aus Lactose und Sojaprotein in der Weise, daß sich eine Mischungstemperatur von etwa 35°C bis 80°C einstellt.

Dabei wird die Lösungsmittelkomponente in die wäßrige Phase überführt und die hydrophobe Phase des/der Carotinoid(e) entsteht als nanodisperse Phase.

Hinsichtlich einer näheren Verfahrens- und Apparatebeschreibung zur oben genannten Dispergierung wird an dieser Stelle auf EP B-0 065 193 Bezug genommen.

Die Überführung in ein Trockenpulver kann dabei u.a. durch Sprühtrocknung, Sprühkühlung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Zur Erhöhung der mechanischen Stabilität des Endproduktes kann es in einigen Fällen zweckmäßig sein, dem Kolloid einen weiteren Weichmacher zuzusetzen, wie Zucker oder Zuckeralkohole, z.B. Saccharose, Glucose, Glucosesirup, Dextrin, Invertzucker, Sorbit, Mannit oder Glycerin.

Zur Erhöhung der Stabilität des Wirkstoffes gegen oxidativen Abbau ist es vorteilhaft, Stabilisatoren wie $\alpha$-Tocopherol, t-Butyl-hydroxy-toluol, t-Butylhydroxyanisol, Ascorbinsäure oder Ethoxyquin zuzusetzen. Sie können entweder der wäßrigen oder der Lösungsmittel-Phase zugesetzt werden, vorzugsweise werden sie jedoch gemeinsam mit den Wirkstoffen in der Lösungsmittel-Phase gelöst.

Unter Umständen kann es auch vorteilhaft sein, zusätzlich in der Lösungsmittel-Phase ein physiologisch zugelassenes Ö1 wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.-%, besonders bevorzugt 20 bis 100 Gew.-%, bezogen auf das/die Xanthophyll(e), zu lösen, das dann gemeinsam mit den Wirkstoffen und den genannten Zusatzstoffen beim Mischen mit der wäßrigen Phase extrem feinteilig ausgefällt wird.

Das Verhältnis Schutzkolloid und Lactose zu Carotinoid wird im allgemeinen so gewählt, daß ein Endprodukt erhalten wird, das zwischen 0,1 und 30 Gew.-%, bevorzugt 1 bis 25 Gew.-%, besonders bevorzugt 5 bis 20 Gew.-% Carotinoid, 10 bis 70 Gew.-% eines Schutzkolloids, 10 bis 70 Gew.-% Lactose, alle Prozentangaben bezogen auf die Trockenmasse des Pulvers, sowie gegebenenfalls geringe Mengen eines Stabilisators enthält.

Die Erfindung betrifft auch Trockenpulver von Carotinoiden, erhältlich nach einem der eingangs genannten Verfahren.

Bevorzugt handelt es sich dabei um Trockenpulver, enthaltend Sauerstoff-haltige Carotinoide, ausgewählt aus der Gruppe, bestehend aus Astaxanthin, Canthaxanthin, Lutein, Zeaxanthin, Citranaxanthin und $\beta$-Apo-8'-Carotinsäureethylester, besonders bevorzugt Canthaxanthin und Astaxanthin, ganz besonders bevorzugt Astaxanthin.

Der Wirkstoffgehalt in den erfindungsgemäßen Trockenpulvern liegt im Bereich von 0,1 bis 30 Gew.-%, bevorzugt 1 bis 25 Gew.-%, besonders bevorzugt 5 bis 20 Gew.-%, ganz besonders bevorzugt im Bereich von 8 bis 15 Gew.-%.

Der Gehalt an Astaxanthin und/oder Canthaxanthin in den erfindungsgemäßen Zubereitungen liegt bevorzugt im Bereich von 5 bis 20 Gew.-%.

Die erfindungsgemäßen Trockenpulver zeichnen sich u.a. dadurch aus, daß sie sich in wäßrigen Systemen unter Erzielung einer gleichmäßigen Feinverteilung des Wirkstoffes im Korngrößenbereich kleiner 1 $\mu$m problemlos wieder redispergieren lassen.

Die Verwendung einer Kombination aus Lactose und Sojaprotein als Formulierhilfsstoffe hat gegenüber Zuckern, beispielsweise Glucose, den Vorteil, daß die damit hergestellten Carotinoid-Formulierungen eine besonders gut Farbstärke verbunden mit einer verbesserten Bioverfügbarkeit zeigen.

Die nach dem erfindungsgemäßen Verfahren erhältlichen Trockenpulver weisen eine höhere Schüttdichte auf als vergleichbare, beispielsweise mit Glucosesirup hergestellte Formulierungen. Überraschenderweise wurde gefunden, daß die höhere Schüttdichte gleichzeitig zu einer höheren Stabilität der erfindungsgemäßen Formulierungen beiträgt.

Darüberhinaus wurde gefunden, daß man kolloidal stabile und nicht vernetzende nanopartikuläre Wirkstoffdispersionen von Sauerstoffhaltigen Carotinoiden erhält, deren Viskositätsverhalten annähernd denen von Newtonschen Flüssigkeiten entspricht. Derartige Flüssigkeiten zeichnen sich dadurch aus, daß deren durch die Newtonsche Gleichung $\tau = h \cdot D$ definierter Fließwiderstand bei gegebener Temperatur eine Stoffkonstante ist ($\tau$ = Schubspannung, D = Schergefälle, h = dynamische Viskosität). Die graphische Darstellung des Fließverhaltens newtonscher Flüssigkeiten ergibt bei gegebener Temperatur annähernd eine Gerade. Insbesondere ändert sich die Viskosität der Wirkstoffdispersion bei 40°C und bei 60°C im Scherbereich zwischen $10^{-2}$ sec$^{-1}$ und $10^{+2}$ sec$^{-1}$ um weniger als $\pm 50\%$.

Die Vorteile dieses annähernd newtonschen Viskositätsverhaltens liegen u.a. darin, daß sich die Wirkstoffdispersionen, insbesondere nach der Aufkonzentrierung leichter Pumpen lassen, als dies bei strukturviskosen Dispersionen der Fall ist. Beim Sprühtrocknen haben die annähernd newtonschen Wirkstoffdispersionen außerdem den Vorteil, daß sich die Parameter des Sprühkopfes leichter optimieren lassen und daß sich diese Dispersionen im Sprühkopf unkritischer verhalten.

Bei der Verwendung eines Gemisches aus teilabgebauten Sojaproteinen mit einem bevorzugten Abbaugrad > 5% und Lactose, lassen sich Xanthophyll-haltige Trockenpulver mit der bereits genannten, verbesserten Farbstärke sowie außerdem einer verbesserter Kaltwasser-Redispergierbarkeit herstellen.

Teilabgebaute Sojaproteine mit einem Abbaugrad größer 5 % zeigen überraschenderweise eine bessere Verträglichkeit mit den eingangs genannten wassermischbaren Lösungsmitteln. Dadurch sind konzentriertere Fahrweisen und somit ein wirtschaftlicheres Verfahren zur Herstellung der erfindungsgemäßen Trockenpulver möglich.

Ferner konnte beobachtet werden, daß mit dem erfindungsgemäßen Verfahren die Zusammenlagerung der Xanthophylle zu H-Aggregaten vermieden wird.

Die Aggregation von Carotinoiden ist ein in der Literatur bereits bekanntes und in zahlreichen Publikationen beschriebenes Phänomen [P. Song, T. A. Moore, Photochemistry and Photobiology, 19, 435-441 (1974); A. V. Ruban, P. Horton, A. J. Young, J. Photochem. Photobiol. B: Biol., 21, 229-234 (1993); V. R. Salares, N.M. Young, P. R. Carey, H. J. Bernstein, Journal of Raman Spectroscopy, 6(6), 282-288 (1977)].

Carotinoid-Aggregate können beispielsweise dadurch erzeugt werden, daß man eine Lösung eines Carotinoids in einem wassermischbaren, organischen Lösungsmittel wie z.B. Isopropanol, Ethanol, Aceton oder Tetrahydrofuran mit Wasser vermischt.

So können, wie in der oben genannten Literatur beschrieben, bei Wahl der richtigen Mengenverhältnisse von Wasser und organischem Lösungsmittel entweder sogenannte H- oder J-Aggregate erzeugt werden.

Unter H-Aggregaten versteht man eine "kartenspielähnliche" Stapelung der Polyenketten (card-stack aggregate), die sich im UV/Vis-Spektrum durch das Auftreten einer neuen, im Vergleich zur Absorption der monomer vorliegenden Formen hypsochrom verschobenen Bande im Bereich zwischen 320 und 400 nm charakterisieren läßt. J-Aggregate dagegen stellen entweder eine lineare Kopf-Schwanz Verknüpfung (head-tail aggregates) der Polyene dar oder sie sind fischgrätenartig angeordnet (herringbone aggregates). Beide Anordnungen bewirken eine bathochrome Verschiebung der UV-Absorption der Polyene.

Fütterungsversuche an Forellen haben gezeigt, daß H-Aggregate von Xanthophyllen, insbesondere die H-Aggregate von Astaxanthin eine schlechtere Bioverfügbarkeit aufweisen als die entsprechenden J-Aggregate, was sich als ein weiterer Vorteil der nach dem erfindungsgemäßen Verfahren hergestellten Trockenpulver darstellt.

Die oben genannten Trockenpulver eignen sich insbesondere als Zusatz zu Lebens- und Tierfuttermitteln sowie als Zusatz zu pharmazeutischen Zubereitungen. Typische Einsatzgebiete für die Carotinoid-haltigen Trockenpulver im Tierfuttermittelbereich sind beispielsweise die Fischpigmentierung in der Aquakultur sowie die Eidotter- und Broilerhautpigmentierung in der Geflügelzucht.

In den nachfolgenden Beispielen wird die Durchführung des erfindungsgemäßen Verfahrens näher erläutert.

Beispiel 1

Herstellung eines Astaxanthin Trockenpulvers

In einer beheizbaren Vorlage wurden 48 g kristallines Astaxanthin, 1,6 g Ascorbylpalmitat und 20 g $\alpha$-Tocopherol in 350 g eines azeotropen Isopropanol/Wasser-Gemischs bei Raumtemperatur suspendiert. Die Wirkstoffsuspension wurde dann auf 90°C erwärmt und bei einer Flussrate von 2,1 kg/h kontinuierlich mit weiterem Isopropanol/Wasser-Azeotrop der Temperatur 223°C und einer Flussrate von 2,7 kg/h vermischt, wobei sich Astaxanthin bei einer sich einstellenden Mischungstemperatur von 165°C bei einem Druck von 55 bar auflöste. Diese Wirkstofflösung wurde unmittelbar anschließend mit einer wässrigen Phase, bestehend aus einer Lösung von 91 g teilabgebautem Sojaprotein mit einem Abbaugrad von 7 %, 182 g Lactose in 10540 g destilliertem Wasser, in dem der pH-Wert mit 1 M NaOH auf pH 9,5 eingestellt wurde, bei einer Flussrate von 60 kg/h vermischt.

Die bei der Mischung entstandenen Wirkstoffteilchen wiesen im Isopropanol/Wasser-Gemisch eine Teilchengröße von 132 nm auf, bei einem E1/1-Wert[1] von 127.

[1] Der E1/1-Wert definiert in diesem Zusammenhang die spezifische Extinktion einer 1 %igen wäßrigen Dispersion eines 10 gew.-%igen Trockenpulvers in einer 1 cm-Küvette im Absorptionsmaximum.

Anschließend wurde die Wirkstoff-Suspension am Dünnfilmverdampfer auf eine Konzentration von ca. 3,9 % Wirkstoffgehalt aufkonzentriert und sprühgetrocknet. Das Trockenpulver wies einen Astaxanthin-Gehalt von 12,8 Gew.-% und ein Schüttgewicht von 430 g/l auf. Das in Wasser redispergierte Trockenpulver hatte eine Teilchengröße von 181 nm und wies einen E1/1-Wert von 126 auf.

Vergleichsbeispiel

Astaxanthin Trockenpulver unter Verwendung einer Kombination aus Sojaprotein und Glucosesirup

In einer beheizbaren Vorlage wurden 48 g kristallines Astaxanthin, 1,6 g Ascorbylpalmitat und 20 g $\alpha$-Tocopherol in 350 g eines azeotropen Isopropanol/Wasser-Gemischs bei Raumtemperatur suspendiert. Die Wirkstoffsuspension wurde dann auf 90°C erwärmt und bei einer Flussrate von 2,1 kg/h kontinuierlich mit weiterem Isopropanol/Wasser-Azeotrop der Temperatur 223°C und einer Flussrate von 2,7 kg/h vermischt, wobei sich Astaxanthin bei einer sich einstellenden Mischungstemperatur von 167°C bei einem Druck von 55 bar auflöste. Diese Wirkstofflösung wurde unmittelbar anschließend mit einer wässrigen Phase, bestehend aus einer Lösung von 91 g teilabgebautem Sojaprotein mit einem Abbaugrad von 7 %, 182 g Glucosesirup in 10540 g destilliertem Wasser, in dem der pH-Wert mit 1 M NaOH auf pH 9,5 eingestellt wurde, bei einer Flussrate von 60 kg/h vermischt.

Die bei der Mischung entstandenen Wirkstoffteilchen wiesen im Isopropanol/Wasser-Gemisch eine Teilchengröße von 146 nm auf, bei einem E1/1-Wert von 125.

Anschließend wurde die Wirkstoff-Suspension am Dünnfilmverdampfer auf eine Konzentration von ca. 3,6 % Wirkstoffgehalt aufkonzentriert und sprühgetrocknet. Das Trockenpulver wies einen Astaxanthin-Gehalt von 12,5 Gew.-% und ein Schüttgewicht von 400 g/l auf. Das in Wasser redispergierte Trockenpulver hatte eine Teilchengröße von 347 nm und wies einen E1/1-Wert von 101 auf.

Beispiel 3

Canthaxanthin Trockenpulver

Zunächst wurden 48 g kristallines Canthaxanthin, 4 g Ascorbylpalmitat und 16 g $\alpha$-Tocopherol in 350 g eines azeotropen Isopropanol/Wasser-Gemischs bei Raumtemperatur suspendiert. Diese Wirkstoffsuspension wurde dann auf 88°C erwärmt und bei einer Flussrate von 2,9 kg/h kontinuierlich mit weiterem heißem Isopropanol/Wasser-Azeotrop bei einer Flussrate von 4,8 kg/h vermischt, wobei sich Canthaxanthin bei einer sich einstellenden Mischungstemperatur von 175°C und bei einem Druck von 55 bar auflöste. Diese Wirkstofflösung wurde sodann mit einer wässrigen Phase, bestehend aus einer Lösung von 106 g teilabgebautem Sojaprotein mit einem Abbaugrad von 7 % und 219 g Lactose in 7050 g destilliertem Wasser, in dem der pH-Wert mit 1 M NaOH auf pH 9,5 eingestellt worden war, bei einer Flussrate von 52 kg/h vermischt.

Die bei der Mischung entstandenen Wirkstoffteilchen wiesen im Isopropanol/Wasser-Gemisch eine Teilchengröße von 134 nm auf, bei einem E1/1-Wert von 133. Anschließend wurde diese Wirkstoff-Dispersion am Dünnfilmverdampfer auf eine Konzentration von ca. 4,2 % Wirkstoffgehalt aufkonzentriert und sprühgetrocknet. Das Trockenpulver wies einen Canthaxanthin-Gehalt von 12,4 Gew.-% auf. Das in Wasser redispergierte Trockenpulver hatte eine mittlere Teilchengröße von 264 nm und wies einen E1/1-Wert von 121 auf.

**Patentansprüche**

1. Verfahren zur Herstellung von Trockenpulvern eines oder mehrerer Carotinoide durch

   a) Dispergieren eines oder mehrerer Carotinoide in einer wäßrigen molekulardispersen oder kolloiddispersen Lösung eines Gemisches aus Lactose und einem Schutzkolloid und

   b) Überführung der gebildeten Dispersion in ein Trockenpulver durch Abtrennung des Wassers und gegebenenfalls zusätzlich verwendeter Lösungsmittel und Trocknung, gegebenenfalls in Gegenwart eines Überzugsmaterials,

   **dadurch gekennzeichnet, daß** man als Schutzkolloid im Verfahrensschritt a) mindestens ein Sojaprotein verwendet.

2. Verfahren nach Anspruch 1, **dadurch gekennzeichnet, daß** es sich beim Dispergierschritt a) um die Herstellung einer Suspension eines oder mehrerer Carotinoide in einer wäßrigen molekulardispersen oder kolloiddispersen Lösung eines Gemisches aus Lactose und mindestens einem Sojaprotein handelt.

3. Verfahren nach Anspruch 2, **dadurch gekennzeichnet, daß** man die im Verfahrensschritt a) hergestellte Suspension vor der Überführung in ein Trockenpulver mahlt.

**4.** Verfahren nach Anspruch 1, **dadurch gekennzeichnet, daß** das Dispergieren in der Stufe a) folgende Schritte umfaßt:

a$_1$) Lösen eines oder mehrerer Carotinoide in einem mit Wasser mischbaren, organischen Lösungsmittel oder in einer Mischung aus Wasser und einem mit Wasser mischbaren, organischen Lösungsmittel oder

a$_2$) Lösen eines oder mehrerer Carotinoide in einem mit Wasser nicht mischbaren, organischen Lösungsmittel und

a$_3$) Mischen der nach a$_1$) oder a$_2$) erhaltenen Lösung mit einer wäßrigen molekulardispersen oder kolloiddispersen Lösung eines Gemisches aus Lactose und mindestens einem Sojaprotein, wobei die hydrophobe Phase des Carotinoids als nanodisperse Phase entsteht.

**5.** Verfahren nach einem der Ansprüche 1 bis 4, **dadurch gekennzeichnet, daß** man als Schutzkolloid mindestens ein teilabgebautes Sojaprotein mit einem Abbaugrad von 0,1 bis 20 % verwendet.

**6.** Verfahren nach einem der Ansprüche 1 bis 5, **dadurch gekennzeichnet, daß** es sich bei den verwendeten Carotinoiden um Sauerstoff-haltige Carotinoide handelt.

**7.** Verfahren nach Anspruch 6, **dadurch gekennzeichnet, daß** es sich bei den Sauerstoff-haltigen Carotinoiden um Verbindungen, ausgewählt aus der Gruppe, bestehend aus Astaxanthin, Canthaxanthin, Lutein, Zeaxanthin, Citranaxanthin und β-Apo-8'-Carotinsäureethylester handelt.

**8.** Verfahren nach Anspruch 7, **dadurch gekennzeichnet, daß** man

a) Astaxanthin und/oder Canthaxanthin in einem mit Wasser mischbaren, organischen Lösungsmittel oder einer Mischung aus Wasser und einem mit Wasser mischbaren, organischen Lösungsmittel bei Temperaturen größer 30°C löst,

b) die erhaltene Lösung mit einer wäßrigen molekulardispersen oder kolloiddispersen Lösung eines Gemisches aus Lactose und einem teilabgebauten Sojaprotein mit einem Abbaugrad von 0,1 bis 20 % mischt und

c) die gebildete Dispersion in ein Trockenpulver überführt.

**9.** Verfahren nach Anspruch 8, **dadurch gekennzeichnet, daß** man als Carotinoid Astaxanthin verwendet.

**10.** Carotinoid-haltige Trockenpulver, erhältlich nach einem Verfahren, definiert gemäß einem der Ansprüche 1 bis 9.

**11.** Trockenpulver nach Anspruch 10 mit einem Carotinoidgehalt von 0,1 bis 30 Gew.-%.

**12.** Trockenpulver nach einem der Ansprüche 10 oder 11, enthaltend Sauerstoff-haltige Carotinoide, ausgewählt aus der Gruppe, bestehend aus Astaxanthin, Canthaxanthin, Lutein, Zeaxanthin, Citranaxanthin und β-Apo-8'-Carotinsäureethylester.

**13.** Trockenpulver nach Anspruch 12, enthaltend 5 bis 20 Gew.-% Astaxanthin.

**14.** Trockenpulver nach Anspruch 12, enthaltend 5 bis 20 Gew.-% Canthaxanthin.

**15.** Verwendung der Carotinoid-haltigen Trockenpulver, definiert gemäß einem der Ansprüche 10 bis 14, als Zusatz zu Lebensmitteln, Pharmazeutika und/oder Tierfuttermitteln.

**Claims**

**1.** A process for producing dry powders of one or more carotenoids by

a) dispersing one or more carotenoids in an aqueous molecular or colloidal solution of a mixture of lactose and a protective colloid and

b) converting the dispersion which has formed into a dry powder by removing the water and, if appropriate, additionally used solvents and drying, if appropriate in the presence of a coating material,

wherein at least one soybean protein is used as protective colloid in process step a).

**2.** The process according to claim 1, wherein the dispersion step a) comprises the preparation of a suspension of one or more carotenoids in an aqueous molecular or colloidal solution of a mixture of lactose and at least one soybean protein.

**3.** The process according to claim 2, wherein the suspension prepared in process step a) is ground before conversion into a dry powder.

**4.** The process according to claim 1, wherein the dispersion in stage a) comprises the following steps:

$a_1$) dissolving one or more carotenoids in a water-miscible organic solvent or in a mixture of water and a water-miscible organic solvent or
$a_2$) dissolving one or more carotenoids in a water-immiscible organic solvent and
$a_3$) mixing the solution obtained as in $a_1$) or $a_2$) with an aqueous molecular or colloidal solution of a mixture of lactose and at least one soybean protein, resulting in the hydrophobic phase of the carotenoid as nanodisperse phase.

**5.** The process according to any of claims 1 to 4, wherein at least one partially degraded soybean protein with a degree of hydrolysis of from 0.1 to 20% is used as protective colloid.

**6.** The process according to any of claims 1 to 5, wherein the carotenoids used are oxygen-containing carotenoids.

**7.** The process according to claim 6, wherein the oxygen-containing carotenoids are compounds selected from the group consisting of astaxanthin, canthaxanthin, lutein, zeaxanthin, citranaxanthin and ethyl β-apo-8'-carotenoate.

**8.** The process according to claim 7, wherein

a) astaxanthin and/or canthaxanthin is dissolved in a water-miscible organic solvent or a mixture of water and a water-miscible organic solvent at temperatures above 30°C,
b) the resulting solution is mixed with an aqueous molecular or colloidal solution of a mixture of lactose and a partially degraded soybean protein with a degree of hydrolysis of from 0.1 to 20%, and
c) the dispersion which has formed is converted into a dry powder.

**9.** The process according to claim 8, wherein astaxanthin is used as carotenoid.

**10.** A carotenoid-containing dry powder obtainable by a process as defined in any of claims 1 to 9.

**11.** The dry powder according to claim 10 with a carotenoid content of from 0.1 to 30% by weight.

**12.** The dry powder according to either of claims 10 or 11, comprising oxygen-containing carotenoids selected from the group consisting of astaxanthin, canthaxanthin, lutein, zeaxanthin, citranaxanthin and ethyl β-apo-8'-carotenoate.

**13.** The dry powder according to claim 12, comprising 5 to 20% by weight of astaxanthin.

**14.** The dry powder according to claim 12, comprising 5 to 20% by weight of canthaxanthin.

**15.** The use of the carotenoid-containing dry powders as defined in any of claims 10 to 14 as addition to human foods, pharmaceuticals and/or animal feeds.

**Revendications**

**1.** Procédé de préparation de poudres sèches d'un ou de plusieurs caroténoïdes par

a) dispersion d'un ou de plusieurs caroténoïdes dans une solution aqueuse dispersée à l'état moléculaire ou dispersée à l'état colloïdal d'un mélange de lactose et d'un colloïde de protection, et
b) transformation de la dispersion formée en une poudre sèche par isolement de l'eau et éventuellement du solvant utilisé en supplément et séchage, éventuellement en présence d'une matière de revêtement,

**caractérisé en ce que**, comme colloïde de protection dans l'étape a) du procédé, on utilise au moins une protéine de soja.

2. Procédé suivant la revendication 1, **caractérisé en ce que**, pour ce qui concerne l'étape de dispersion a), il s'agit de la préparation d'une suspension d'un ou de plusieurs caroténoïdes dans une solution aqueuse dispersée à l'état moléculaire ou à l'état colloïdal d'un mélange de lactose et d'au moins une protéine de soja.

3. Procédé suivant la revendication 2, **caractérisé en ce qu'**on broie la suspension préparée dans l'étape a) du procédé avant la transformation en une poudre sèche.

4. Procédé suivant la revendication 1, **caractérisé en ce que** la dispersion dans l'étape a) comporte les phases suivantes :

   $a_1$) une dissolution d'un ou de plusieurs caroténoïdes dans un solvant organique miscible à l'eau ou dans un mélange d'eau et d'un solvant organique miscible à l'eau, ou
   $a_2$) une dissolution d'un ou de plusieurs caroténoïdes dans un solvant organique non miscible à l'eau, et
   $a_3$) un mélange de la solution obtenue selon $a_1$) ou $a_2$) avec une solution aqueuse dispersée à l'état moléculaire ou à l'état colloïdal d'un mélange de lactose et d'au moins une protéine de soja, la phase hydrophobe du caroténoïde étant obtenue sous la forme d'une phase nanodispersée.

5. Procédé suivant l'une des revendications 1 à 4, **caractérisé en ce que**, comme colloïde de protection, on utilise au moins une protéine de soja partiellement dégradée, présentant un degré de dégradation de 0,1 à 20 %.

6. Procédé suivant l'une des revendications 1 à 5, **caractérisé en ce que**, pour ce qui concerne les caroténoïdes utilisés, il s'agit de caroténoïdes contenant de l'oxygène.

7. Procédé suivant la revendication 6, **caractérisé en ce que**, pour ce qui concerne les caroténoïdes contenant de l'oxygène, il s'agit de composés choisis parmi le groupe constitué d'astaxanthine, de canthaxanthine, de lutéine, de zéaxanthine, de citranaxanthine et de β-apo-8'-caroténate d'éthyle.

8. Procédé suivant la revendication 7, **caractérisé en ce que**

   a) on dissout de l'astaxanthine et/ou de la canthaxanthine dans un solvant organique miscible à l'eau ou un mélange d'eau et d'un solvant organique miscible à l'eau, à des températures supérieures à 30°C,
   b) on mélange la solution obtenue avec une solution aqueuse dispersée à l'état moléculaire ou à l'état colloïdal d'un mélange de lactose et d'une protéine de soja partiellement dégradée, présentant un degré de dégradation de 0,1 à 20 %, et
   c) on transforme la dispersion formée en une poudre sèche.

9. Procédé suivant la revendication 8, **caractérisé en ce que**, comme caroténoïde, on utilise de l'astaxanthine.

10. Poudre sèche contenant du caroténoïde, que l'on peut obtenir suivant un procédé défini suivant l'une des revendications 1 à 9.

11. Poudre sèche suivant la revendication 10, présentant une teneur en caroténoïde de 0,1 à 30 % en poids.

12. Poudre sèche suivant l'une des revendications 10 ou 11, contenant des caroténoïdes contenant de l'oxygène, choisis parmi le groupe constitué d'astaxanthine, de canthaxanthine, de lutéine, de zéaxanthine, de citranaxanthine et de β-apo-8'-caroténate d'éthyle.

13. Poudre sèche suivant la revendication 12, contenant 5 à 20 % en poids d'astaxanthine.

14. Poudre sèche suivant la revendication 12, contenant 5 à 20 % en poids de canthaxanthine.

15. Utilisation de la poudre sèche contenant du caroténoïde, définie suivant l'une des revendications 10 à 14, comme additif pour des produits alimentaires, des produits pharmaceutiques et/ou des aliments pour animaux.